# EUROPEAN PATENT APPLICATION

(11) **EP 4 635 469 A1**
(43) Date of publication of application: **22.10.2025**
(21) Application number: 25165507.2
(22) Date of filing: 24.03.2025
(51) Int. Cl.: A61K 6/30, A61K 6/889, A61K 6/76

(54) **DENTAL GLASS IONOMER CEMENT COMPOSITION**

(30) Priority: 23.03.2024 JP 2024047379
(71) Applicant: Shofu Inc., Kyoto-shi, Kyoto 605-0983 (JP)
(72) Inventor: ITAGAKI, Takuma, Kyoto, 605-0983 (JP); SAKAMOTO, Shuji, Kyoto, 605-0983 (JP); KIMOTO, Katsuya, Kyoto, 605-0983 (JP)
(74) Representative: Eisenführ Speiser

(57) **Abstract**

To provide a dental glass ionomer cement composition that exhibits little stringiness of mixed material, becomes soon after completion of mixing in a state that it is possible to perform shaping operation, is excellent in cavity filling property and is excellent in application property to a dental prosthesis device, and also exhibits excellent mixability and mechanical characteristic.

To provide a dental glass ionomer cement composition comprising (a) acid-reactive glass powder, (b) polyalkenoic acid, (c) water, and (d) porous inorganic filler: 0.075 % by mass or more and 15 % by mass or less, wherein the center portion of the (d) porous inorganic filler is an inorganic particle consisting of only silicon dioxide or consisting of silicon dioxide and an oxide containing one or more metal elements.

## Description

### BACKGROUND OF THE INVENTION

### Field of the Invention

The present invention relates to a dental glass ionomer cement composition for filling and repairing a tooth in which a form was partially lost by mainly caries, breakages and the like or for adhering or luting a dental prosthesis device to a tooth in which a form was partially lost.

### Description of the Related Art

In a dental practice, in order to restore aesthetically and functionally the tooth in which a form was partially lost by caries, breakages and the like, a direct restoration in which a filling material is filled into the tooth and an indirect restoration in which a dental prosthesis device is bonded and/or adhered to a tooth by using a luting material has been performed. One of the representative the filling material and the luting material includes a dental glass ionomer cement, and its greatest feature is its sustained release property of fluoride ions, which strengthens tooth substance and inhibits secondary caries.

A dental glass ionomer cement contains acid-reactive glass powder represented by fluoroaluminosilicate glass powder, polyalkenoic acid and water as the main component, and are generally in the form of two components, such as a powder-liquid type or a two-paste type. In the dental glass ionomer cement in each forms of these, a mixed material is prepared immediately before use by performing a hand mixing or a mechanical mixing in the case of the powder-liquid type and by performing a hand mixing or an automatic mixing using a mixing tip in the case of two-paste type, to use. In the dental glass ionomer cement mixed in this manner, an acidic compound such as a polyalkenoic acid acts on an acid-reactive glass powder in the presence of water, causing polyvalent metal ions (Al³⁺, Ca²⁺, Sr²⁺, etc.) to elute from the acid-reactive glass powder, and the eluted polyvalent metal ions form ionic bonds (acid-base reaction) with the acidic groups of the polyalkenoic acid, forming a crosslinked structure between the polyalkenoic acids via the polyvalent metal ions, and the cement sets.

Various techniques to improve the property of the dental glass ionomer cement by adding additives to the main components have been proposed. For example, Japanese Unexamined Patent Application Publication No. 2019-137618 A discloses a technique for improving the transparency of the set product by adding a polyvalent metal compound to a dental glass ionomer cement composition.

Furthermore, Japanese Unexamined Patent Application Publication No. 2023-9286 A discloses a technique for improving mechanical property by adding a water-reducing agent to a dental glass ionomer cement composition.

### SUMMARY OF THE INVENTION

### Technical Problem

Since a dental glass ionomer cement contains a high consistency aqueous solution of polyalkenoic acid in its composition, there is a tendency that the mixed material is stringy immediately after mixing. This stringiness can adversely affect the operability of filling and bonding, and the filling operation is particularly affected with this. For example, after filling a mixed material immediately after mixing is filled into a cavity using a dental instrument such as a dental syringe or an instrument, when the dental instrument is pulled away from the mixed material, there is a case where the mixed material becomes stringy to adhere to the surrounding tooth substance or oral mucosa. In such a case, it is necessary to carefully remove the adhesion, which makes the operation complicated.

In addition, since the dental glass ionomer cement begins to set due to the acid-base reaction immediately after mixing, regardless of the properties of the mixed material, after a certain period of time, the stringiness of the mixed material decreases, making it easier to perform shaping operations. Therefore, when shaping is performed on the mixed material filled in the cavity, the shaping operation is performed after waiting until the stringiness of the mixed material is reduced. On the other hand, if the mixed material is extremely stringy and it takes time for the stringiness to decrease, the treatment time is longer and the risk of the treatment site being contaminated by saliva increases.

As the method for reducing stringiness of the mixed material immediately after mixing, there is a method for increasing the ratio or the particle diameter of acid-reactive glass powder contained in the dental glass ionomer cement. However, when the ratio of the acid-reactive glass powder is increased, the viscosity of the mixed material increases, and thus there is a case where the mixability decreases. Furthermore, when the particle diameter of the acid-reactive glass powder is increased, the acid-base reactivity between the acid-reactive glass powder and the polyalkenoic acid decreases, and thus there is a case where a decrease in the mechanical characteristic of the set product is caused.

Therefore, an object of the present invention is to provide a dental glass ionomer cement composition which causes less stringiness of the mixed material than conventional techniques, becomes soon after completion of mixing in a state that it is possible to perform shaping operation, is excellent in cavity filling property and in application property to a dental prosthesis device, and also exhibits excellent mixability and mechanical characteristic.

### Solution to Problem

The present inventors made an intensive study under the above problems. As a result, the present inventors have found that, by compounding a specific porous inorganic filler in a specific range, the dental glass ionomer cement composition exhibits little stringiness and therefore is excellent in cavity filling property and is excellent in application property to a dental prosthesis device, and becomes soon after completion of mixing in a state that it is possible to perform shaping operation, and also exhibits excellent mixability and mechanical characteristic, and the present invention has been completed.

That is, the above problem can be solved by the following component composition.
A dental glass ionomer cement composition comprising
(a) acid-reactive glass powder,
(b) polyalkenoic acid,
(c) water, and
(d) porous inorganic filler: 0.075 % by mass or more and 15 % by mass or less, wherein
the center portion of the (d) porous inorganic filler is an inorganic particle consisting of only silicon dioxide or consisting of silicon dioxide and an oxide containing one or more metal elements.

### Advantageous Effects of Invention

The present invention provide a dental glass ionomer cement composition that exhibits little stringiness immediately after mixing and therefore is excellent in cavity filling property and is excellent in application property to a dental prosthesis device, and becomes soon after completion of mixing in a state that it is possible to perform shaping operation and therefore shorten treatment time, and also exhibits excellent mixability and mechanical characteristic.

### DETAILED DESCRIPTION OF THE PREFERRED EMBODIMENTS

In the present invention, the (d) porous inorganic filler may have a 50 % particle diameter (D50) within the range of 0.1 µm or more and 10 µm or less, a pore volume within the range of 0.01 cc/g or more and 1.00 cc/g or less, and a specific surface area within the range of 5 m²/g or more and 500 m²/g or less.

In the present invention, the dental glass ionomer cement composition may comprise
(a) an acid-reactive glass powder: 44 % by mass or more and 80 % by mass or less,
(b) a polyalkenoic acid: 7.5 % by mass or more and 20 % by mass or less,
(c) water: 7 % by mass or more and 32 % by mass or less, and
(d) porous inorganic filler: 0.075 % by mass or more and 15 % by mass or less.

In the present invention, shaping start time of the dental glass ionomer cement composition bay be within 30 seconds.

The present invention will be described in detail below. In the present specification, the term "dental glass ionomer cement composition" means a dental material that does not contain, under the intention of imparting setting through a polymerization reaction, a compound having a polymerizable group, such as a polymerizable monomer, an oligomer having a polymerizable group and/or a polymer having a polymerizable group, and that sets primarily through an acid-base reaction that occurs between an acid-reactive glass powder and a polyalkenoic acid in the presence of water.

In the present specification, "polyalkenoic acid" means a polymer containing an ethylenically unsaturated monomer unit having an acidic group. In the present specification, the term "(meth)acrylate" inclusively refers to both acrylate and methacrylate, the term "(meth)acryloyl" inclusively refers to both acryloyl and methacryloyl.

In the present specification, the degree of stringiness of the mixed material is evaluated by the following method. That is, a mixed material of the dental glass ionomer cement composition (total amount specified as 360 mg) was filled into a simulated cavity of a certain size, the excess was scraped off to make the surface flat, and after 10 seconds from the end of mixing, the cylindrical tip of a metal instrument (diameter: φ1.5 mm) was vertically submerged 0.5 mm into the mixed material, and the instrument was immediately gently pulled up to observe the degree of stringiness. The test is performed under an environment of a temperature of 23±1 °C and a humidity of 50±10%. At this time, when the mixed material does not become stringy or when there is only slight stringiness, it is evaluated as "good mixed material property with little stringiness".

In addition, the degree of stringiness of the mixed material is evaluated using the same method, and the time from the end of mixing to until the mixed material has "good mixed material property with little stringiness" is defined as "start time of shapable".

In addition, in the present specification, the term "50% particle diameter (D50)" means a particle diameter at which an integrated value from the small particle diameter side becomes 50% in a volume-based particle diameter distribution measured using a laser diffraction/scattering type particle size distribution measuring device. The present invention also relates a mixing device for capsules for dental restorative materials, and capsules for mixing and dispensing dental materials. In the dental field, capsules for dental cement have been widely used as container for two-component mixed and kneaded dental cements. When using a capsule for dental cement, the two components are mixed and kneaded using an automatic mixer such as a capsule mixer, and then a filling tool such as an applier is attached to the capsule, and the dental cement inside the capsule can be filled into the application site such as a cavity. In the dental field, capsules for dental cement have been widely used as container for two-component mixed and kneaded dental cements. When using a capsule for dental cement, the two components are mixed and kneaded using an automatic mixer such as a capsule mixer, and then a filling tool such as an applier is attached to the capsule, and the dental cement inside the capsule can be filled into the application site such as a cavity.

In the present specification, the term "pore volume" refers to a value determined by the BJH method from an adsorption isotherm obtained by a nitrogen adsorption method.

In the present specification, the term "specific surface area" refers to a value determined by the BET method from an adsorption isotherm obtained by a nitrogen adsorption method.

When using the dental glass ionomer cement composition of the present invention, for example, a mixing device for capsule for dental restorative material and a capsule for mixing and dispensing dental material can be used. In the dental field, capsules for dental cement have been widely used as container for two-component mixed and mixed dental cements. When using a capsule for dental cement, the two components are mixed and mixed using an automatic mixer such as a capsule mixer, and then a filling tool such as an applier is attached to the capsule, and the mixed material inside the capsule can be filled into the application site such as a cavity.

The dental glass ionomer cement composition of the present invention contains (a) acid-reactive glass powder, (b) polyalkenoic acid, (c) water and specific (d) porous inorganic filler as essential components and contains specific amount of the (d) porous inorganic filler, and by having this component configuration, the mixed material exhibits little stringiness and therefore is excellent in cavity filling property and is excellent in application property to a dental prosthesis device, becomes soon after completion of mixing in a state that it is possible to perform shaping operation, and also exhibits excellent mixability and mechanical characteristic. Hereinafter, the above component of the present invention will be described in detail.

### <(a) Acid-reactive glass powder>

The (a) acid-reactive glass powder that can be used in the dental glass ionomer cement composition of the present invention is a component contributing to the setting of the composition and needs to contain an acid-reactive element such as metal element, and fluorine element. Because the (a) acid-reactive glass powder contains an acid reactive element, the acid-base reaction of the (a) acid-reactive glass powder with the acid group contained in the (b) polyalkenic acid progresses in the presence of (c) water. Specific examples of the acid reactive element include sodium, potassium, calcium, strontium, barium, lanthanum, aluminum and zinc, but are not limited thereto. One or two or more kinds of these acid reactive elements may be contained and a content thereof is not particularly limited.

Further, it is preferable that the (a) acid-reactive glass powder includes an X-ray impermeable element in order to impart X-ray contrast property to the dental glass ionomer cement composition of the present invention. Specific examples of the X-ray impermeable element include strontium, lanthanum, zirconium, titanium, yttrium, ytterbium, tantalum, tin, tellurium, tungsten and bismuth, but are not limited thereto. In addition, other element contained in the (a) acid-reactive glass powder is not particularly limited and the (a) acid-reactive glass powder in the present invention may include various elements.

Examples of the (a) acid-reactive glass powder include aluminosilicate glass, borosilicate glass, aluminoborate glass, boro aluminosilicate glass, phosphate glass, borate glass, silicate glass which contain the above described acid reactive element, fluorine element and X-ray impermeable element, but are not limited thereto.

Further, a particle shape of the (a) acid-reactive glass powder is not particularly limited, but arbitral particle shapes such as spherical, needle-like, plate-like, ground-like, and scaly-shape may be used without any limitation. These (a) acid-reactive glass powder may be used alone or in a combination of a few kinds thereof.

A manufacturing method of the (a) acid-reactive glass powder is not particularly limited, and any of the (a) acid-reactive glass powder manufactured by any manufacturing methods such as a melting method, a vapor phase method and a sol-gel method may be used without any problem. Among them, the (a) acid-reactive glass powder manufactured by a melting method or a sol-gel method which can easily control a kind of element and the content thereof is preferably used.

The (a) acid-reactive glass powder may be ground to use in order to obtain a desirable particle diameter. A grinding method is not particularly limited, but an acid-reactive glass powder obtained by grinding which use any of wet or dry grinding methods may be used. Specifically, the particle diameter may be appropriately adjusted according to the desired property to be imparted to the dental glass ionomer cement composition of the present invention by grounding a raw material glass with a high speed rotating mill such as a hammer mill and a turbo-mill, a container driving type mill such as a ball mill, a planetary mill and a vibration mill, a medium stirring mill such as an attritor and a bead mill and a jet mill and the like.

It is preferable that the 50% particle diameter (D50) of the (a) acid-reactive glass powder is 0.5 µm or more and 20 µm or less, and more preferably 2.5 µm or more and 20 µm or less. The dental glass ionomer cement composition of the present invention may contain only acid-reactive glass powder having the 50% particle diameter (D50) of 0.5 µm or more and 20 µm or less as the (a) acid-reactive glass powder.

When the 50% particle diameter (D50) of the (a) acid-reactive glass powder is less than 0.5 µm, the surface area thereof increases and it becomes impossible to contain the acid-reactive glass powder in a large amount into the composition, therefore there is a case where the mechanical characteristic decreases. Further there is a case where working time is remarkably shortened. When the 50% particle diameter (D50) of the (a) acid-reactive glass powder exceeds 20 µm, there is a case where the mechanical characteristic decreases. In the case of using as a material for filling, the surface of the material after polishing becomes rough, therefore there is a risk of staining in the oral cavity. Further, in case of using as a material for luting, because the film thickness becomes thick, the attached or adhered dental prosthesis device is lifted and therefore there is a case where the intended fit cannot be obtained.

The (a) acid-reactive glass powder may be treated with various surface treatments, heat treatment, aggregating treatment in a liquid phase or a vapor phase and the like to such a range that the acid-base reaction of the (a) acid-reactive glass powder with the (b) polyalkenoic acid is not adversely affected, in order to adjust operability, a setting characteristic, a mechanical characteristic and the like of the dental glass ionomer cement composition of the present invention. These treatments can be performed alone or in a combination of a few kinds thereof, and the order in which the treatments are performed is not particularly limited. Among them, the surface treatment and the heat treatment are preferable because it is easy to control various characteristics and those are superior in productivity.

Specific examples of the surface treatment of the (a) acid-reactive glass powder include washing with an acid such as phosphoric acid or acetic acid, surface treatment with an acidic compound such as tartaric acid or polycarboxylic acid, surface treatment with a fluoride such as aluminum fluoride and surface treatment with a silane compound such as (meth) acryloyloxymethyl trimethoxysilane, 3-(meth) acryloyloxypropyl trimethoxysilane, 8-(meth) acryloyloxyoctyl trimethoxysilane, 3-mercaptopropyl trimethoxysilane, tetramethoxysilane, tetraethoxysilane, partially hydrolyzed oligomer of tetramethoxysilane and partially hydrolyzed oligomer of tetraethoxysilane. The surface treatment is not limited the above described surface treatment and these surface treatments can be used alone or in a combination thereof. Furthermore, the amount of the surface treatment agent relative to the (a) acid-reactive glass powder when performing the surface treatment is not particularly limited, and may be appropriately adjusted depending on the particle diameter of the (a) acid-reactive glass powder and desired property.

Specific examples of the heat treatment of the (a) acid-reactive glass powder include a treatment method which includes heating for a range of 1 hour to 72 hours within a range of 200 °C or more to 800 °C or less using an electric furnace. The heat treatment which can be used in the present invention is not limited the above described treatment and the treatment process may be a processing at uni-temperature or a multi-stage processing at multi-temperatures.

It is preferable that a content of the (a) acid-reactive glass powder is 44 % by mass or more to 80 % by mass or less mass% and more preferably 63 % by mass or more and 80 % by mass or less based on the whole of the dental glass ionomer cement composition of the present invention. When the content of the (a) acid-reactive glass powder is less than 44 % by mass, there is a case where the mechanical characteristic decrease. Furthermore, when the content of the (a) acid-reactive glass powder exceeds % by mass, there is a case where operability is adversely affected such as a case where working time becomes remarkably shorter or a case where the viscosity of the mixed material increases, resulting in poor mixing property.

### <(b) Polyalkenoic Acid>

The (b) polyalkenoic acid that can be used in the dental glass ionomer cement composition of the present invention is a component contributing to the setting of the composition. Any polyalkenoic acids can be used without any limitation as the (b) polyalkenoic acid, as long as it is a homopolymer or copolymer of an ethylenically unsaturated monomer having at least one or more carboxy groups in the molecule such as an ethylenically unsaturated monocarboxylic acid, an ethylenically unsaturated dicarboxylic acid, an ethylenically unsaturated tricarboxylic acid and the like. Further, the (b) polyalkenoic acid may be a copolymer of an ethylenically polymerizable monomer having no carboxy group and an ethylenically unsaturated monomer having a carboxy group, in the molecule, without any problems.

In such a copolymer, the content of ethylenically unsaturated monomer units having a carboxy group is preferably 60 % or more, more preferably 70 % or more, and most preferably 80 % or more.

Specific examples of the ethylenically unsaturated monomer having a carboxy group that can be used to obtain the (b) polyalkenoic acid include ethylenically unsaturated monocarboxylic acid such as acrylic acid, methacrylic acid, 2-chloroacrylic acid, 3-chloroacrylic acid and 2-cyanoacrylic acid; ethylenically unsaturated dicarboxylic acids such as mesaconic acid, maleic acid, maleic anhydride, itaconic acid, itaconic anhydride, fumaric acid, glutaconic acid and citraconic acid; ethylenically unsaturated tricarboxylic acids such as aconitic acid, 1-butene-1,2,4-tricarboxylic acid and 3-butene-1,2,3-tricarboxylic acid, but are not limited thereto. Among them, it is preferable to use the (b) polyalkenoic acid synthesized from only acrylic acid as a starting material or the (b) polyalkenoic acid synthesized from two or more kinds of starting materials such as acrylic acid and maleic acid, acrylic acid and maleic anhydride, acrylic acid and itaconic acid, and acrylic acid and 3-butene-1,2,3-tricarboxylic acid.

The polymerization method used for obtaining various (b) polyalkenic acid is not particularly limited, and a polymer polymerized by any methods such as solution polymerization, suspension polymerization, emulsion polymerization or the like, may be used without any limitation. In addition, as a polymerization initiator and a chain transfer agent which is used at polymerization, a known polymerization initiator and a known chain transfer agent can be used, and the additive amounts thereof may be appropriately adjusted depending on the desired characteristics. The (b) polyalkenic acid obtained by such way can be used alone, or in a combination of a few kinds thereof.

A weight average molecular weight of the (b) polyalkenoic acid is preferably within a range of 30,000 or more and 300,000 or less. Herein, the weight average molecular weight means the average molecular weight which is calculated based on molecular weight distribution measured by gel permeation chromatography. When the weight average molecular weight of the (b) polyalkenoic acid is less than 30,000, there is a case where the mechanical characteristic decreases. In addition, when the weight average molecular weight of the (b) polyalkenoic acid is more than 300,000, there is a case where operability is adversely affected such as a case where the working time becomes remarkably shorter, or there is a case where the viscosity of the mixed material increases, resulting in poor mixing property. The dental glass ionomer cement composition of the present invention may contain only polyalkenoic acid with a weight average molecular weight within a range of 30,000 to 300,000 as the (b) polyalkenoic acid.

In addition, the (b) polyalkenoic acid may be used after some of its carboxy groups have been neutralized with a basic compound for the purpose of adjusting the acid-base reactivity with the (a) acid-reactive glass powder as long as a range that various properties are not adversely affected. Examples of the basic compound used for neutralization include alkali metal hydroxides such as sodium hydroxide, potassium hydroxide and lithium hydroxide; alkali metal carbonates such as sodium carbonate, potassium carbonate and lithium carbonate; and alkali metal hydrogen carbonates such as sodium hydrogen carbonate, potassium hydrogen carbonate and lithium hydrogen carbonate. Furthermore, various amine compounds such as primary amines, secondary amines and tertiary amines may be used without any problem. As the amine compound, triethanolamine, diethanolamine, N-methyldiethanolamine, 2-dimethylaminoethyl (meth) acrylate and the like can be suitably used.

It is preferable that a content of the (b) polyalkenoic acid is 7.5 % by mass or more and 20 % by mass or less and more preferably 10 % by mass or more and 14 % by mass or less based on the whole of the dental glass ionomer cement composition of the present invention. When the content of the (b) polyalkenoic acid is less than 0.5 % by mass, there is a case where the mechanical characteristic decrease. When the content of the (b) polyalkenoic acid is more than 17 % by mass, there is a case where operability is adversely affected such as a case where the working time becomes remarkably shorter or a case where the viscosity of the mixed material increases, resulting in poor mixing property.

### <(c) Water>

The (c) water that can be used in the dental glass ionomer cement composition of the present invention is a component which functions as a solvent for dissolving the (b) polyalkenoic acid, diffuses metal ions eluted from the (a) acid-reactive glass powder, and induces a cross-linking reaction with the (b) polyalkenoic acid.

Any water can be used as the (c) water as long as it does not contain impurities inhibiting the acid-base reaction and adversely affecting on the settability and the mechanical characteristic of the dental glass ionomer cement composition of the present invention without any limitations. Specifically, it is preferably to use distilled water or ion-exchanged water.

It is preferable that a content of the (c) water is 7 % by mass or more and 32 % by mass or less and more preferably 12 % by mass or more and 16 % by mass or less based on the whole of the dental glass ionomer cement composition of the present invention. When the content of the (b) water is less than 0.5 % by mass, there is a case where operability is adversely affected such as a case where working time becomes remarkably shorter or a case where the viscosity of the mixed material increases, resulting in poor mixing property. When the content of the (c) water exceeds 32 % by mass, there is a case where the mechanical characteristic decrease.

### <(d) Porous inorganic filler>

The (d) porous inorganic filler that can be used in the dental glass ionomer cement composition of the present invention is an inorganic filler having at least one or more pores. The presence or absence of pores in an inorganic filler can be measured, for example, by gas adsorption method or mercury intrusion method. More specifically, in the present specification, the (d) porous inorganic filler refers to a filler having a pore volume of 0.01 cc/g or greater as measured by gas adsorption. The dental glass ionomer cement composition of the present invention may contain no fillers other than the (d) porous inorganic filler.

A shape of the (d) porous inorganic filler is not particularly limited, but is preferably spherical or ground-like, because of relatively little effect on the viscosity of the mixed product of the dental glass ionomer cement composition of the present invention. Furthermore, the 50% particle diameter (D50) of the (d) porous inorganic filler is preferably 0.1 µm or more and 10 µm or less, and more preferably 1 µm or more and 8 µm or less. When the 50% particle diameter (D50) of the (d) porous inorganic filler exceeds 10 µm, there is a case where the mechanical property of the dental glass ionomer cement composition of the present invention is reduced. Furthermore, when the 50% particle diameter (D50) is less than 0.1 µm, there is a case where the mixing property and property of the mixed product are adversely affected.

The pore volume of the (d) porous inorganic filler is preferably 0.01 cc/g or more and 1.00 cc/g or less, and more preferably 0.10 cc/g or more and 0.80 cc/g or less. Furthermore, the specific surface area of the (d) porous inorganic filler is preferably 5 m²/g or more and 500 m²/g or less, and more preferably 10 m²/g or more and 300 m²/g or less. Because the (d) porous inorganic filler has such properties, it is possible to effectively reduce stringiness of the mixed product in the dental glass ionomer cement composition of the present invention. When the pore volume and/or specific surface area of the (d) porous inorganic filler is less than the above range, there is a case where the effect of reducing stringiness of the mixed product is difficult to exhibit. Furthermore, when the pore volume and/or specific surface area exceeds the above range, there is a case where the mechanical property is reduced. The dental glass ionomer cement composition of the present invention may contain only a porous inorganic filler having a 50% particle diameter (D50) of 0.1 µm or more and 10 µm or less, a pore volume of 0.01 cc/g or more and 1.00 cc/g or less, and a specific surface area of 5 m/g or more and 500 m/g or less, as the (d) porous inorganic filler.

The (d) porous inorganic filler has at the center portion an inorganic particle consisting of only silicon dioxide or consisting of silicon dioxide and an oxide containing one or more metal elements. Examples of oxides containing metal elements include, but are not limited to, oxides of metal elements such as Al, Ba, Bi, Ca, Ce, Co, Cu, Er, Fe, Hf, Ho, In, La, Mg, Mn, Nd, Ni, Pb, Sb, Sn, Sr, Ta, Ti, Y, Yb, Zn, and Zr. Among these, oxides of Al, Ba, Ca, Co, Cu, Fe, Hf, La, Mg, Ni, Sr, Ti, Zn, and Zr are preferable, and oxides of Ba, Ti, Zr, etc. are more preferable, and oxide of Zr is furthermore preferable. The dental glass ionomer cement composition of the present invention may contain, as the oxide containing a metal element constituting the inorganic particle of the center portion of the (d) porous inorganic filler, only oxides of Al, Ba, Ca, Co, Cu, Fe, Hf, La, Mg, Ni, Sr, Ti, Zn, or Zr or may contain only oxides of Ba, Ti, or Zr, or may contain only oxide of Zr.

The content of the oxide of the metal element contained in the inorganic particles at the center portion of the (d) porous inorganic filler is preferably 30 % by mass or less, calculated as oxide. When the content of the oxide of the metal element exceeds 30% by mass, there is a case where the refractive index of the (d) porous inorganic filler becomes too high, which causes the dental glass ionomer cement composition of the present invention to become opaque.

The (d) inorganic particles at the center portion of the porous inorganic filler may be manufactured, for example, by mixing an acidic silicic acid liquid, a silicon dioxide sol and optionally one or more aqueous metal salt solutions, spray-drying the resulting mixed slurry, and then heat-treating the resulting dried particles. Detailed production methods for the porous inorganic filler are disclosed in, for example, JP 2019-189637 A, but are not limited to these production methods.

Furthermore, in order to adjust the fluidity of the powder material and paste property in the dental glass ionomer cement composition of the present invention, the surface of the inorganic particle of the center portion can be optionally treated with a silane compound such as 3-(meth) acryloyloxymethyl trimethoxysilane, 3-(meth) acryloyloxypropyl trimethoxysilane, 8-(meth) acryloyloxyoctyl trimethoxysilane, 3-mercaptopropyl trimethoxysilane, 3-aminopropyl trimethoxysilane, or 3-glycidoxypropyl trimethoxysilane. Surface treatment agent is not limited to the above described agent, and these surface treatment agents can be used alone or in combination thereof. Furthermore, the amount of surface treatment agent relative to the inorganic particle of the center portion of the (d) porous inorganic filler is not particularly limited and can be adjusted appropriately depending on the particle diameter, pore volume, specific surface area, and desired properties of the inorganic particle of the center portion of the (d) porous inorganic filler.

The (d) porous inorganic filler must be contained within a range of 0.075 % by mass or more and 15 % by mass or less, and more preferably within a range of 3 % by mass or more and 8 % by mass or less, based on the total dental glass ionomer cement composition of the present invention. When the content of the (d) porous inorganic filler is less than 0.075 % by mass, the effect of reducing stringiness of the mixed product is not exhibited. On the other hand, when the content exceeds 15% by mass, the mechanical property is reduced, and the viscosity of the mixed product increases, resulting in poor mixability.

### <Other component>

The dental glass ionomer cement composition of the present invention may arbitrarily contain an acidic compound to such a range that various properties are not influenced, for the purpose of adjusting the working time and set time. Specific examples of these acidic compounds include carboxylic acid compounds such as tartaric acid, citric acid, maleic acid, fumaric acid, malic acid, aconitic acid, tricarballylic acid, itaconic acid, 1-butene-1,2,4-tricarboxylic acid and 3-butene-1,2,3-tricarboxylic acid; phosphoric acid compounds such as phosphoric acid, pyrophosphoric acid and tripolyphosphoric acid; and metal salts of these acidic compounds, but are not limited to thereto. These acidic compounds may be used alone or in a combination of several kinds thereof. When the acidic compound is contained in the dental glass ionomer cement composition of the present invention, the content of the acidic compound is preferably 0.1 % by mass or more and 15 % by mass or less based on the whole of the composition. Further, the dental glass ionomer cement composition of the present invention may not contain an acidic compound.

Further, a surfactant can be optionally contained in the dental glass ionomer cement composition of the present invention to such a range that various properties are not influenced, for the purpose of adjusting the initial compatibility of the powder material and liquid material, or first paste and second paste or mixed material property. The surfactant which can be used in the dental glass ionomer cement composition of the present invention may be any of an ionic surfactant and a nonionic surfactant.

Specific examples of the anionic surfactant in the ionic surfactant include aliphatic carboxylic acid metal salts such as sodium stearate, sulfated aliphatic carboxylic acid metal salts such as sodium dioctyl sulfosuccinate and metal salts of higher alcohol sulfate ester such as sodium stearyl sulfate. In addition, examples of the cationic surfactant include an adduct of higher alkylamine and ethylene oxide, amines made from lower amine, and alkyltrimethylammonium salts such as lauryltrimethylammoniun chloride. Further, examples of the amphoteric surfactant include metal salts of higher alkylaminopropionic acid such as sodium stearylaminopropionate, and betaines such as lauryldimethylbetaine.

Examples of the nonionic surfactant include polyethylene glycol type and polypropylene glycol type in which ethylene oxide or propylene oxide is added to higher alcohols, alkyl phenols, fatty acids, higher fatty amines or aliphatic amides, and polyhydric alcohol type in which polyhydric alcohols, diethanolamines or saccharides is ester bonded to a fatty acid.

The above described surfactant is not limited to these, and may be used alone or in a combination of a few kinds thereof. When the surfactant is contained in the dental glass ionomer cement composition of the present invention, it is preferable that the content of the surfactant is 0.001 % by mass or more and 5 % by mass or less based on the whole of the composition. The dental glass ionomer cement composition of the present invention may not contain a surfactant.

Further, a non acid-reactive powder having no pore can be arbitrarily contained in the dental glass ionomer cement composition of the present invention to such a range that various properties are not influenced, for the purpose of adjusting operability, a mechanical characteristic , or a curing characteristic.

As the non acid-reactive powder having no pore used in the dental glass ionomer cement composition of the present invention, any non acid-reactive powder as long as the non acid-reactive powder does not contain element which may form reaction with an acid group of the non-crosslinked polyalkenic acid can be used without any limitation. Examples of the non acid-reactive powder having no pore include known powder in the dental field such as an inorganic filler, an organic filler and an organic-inorganic complex filler, and these can be used alone or in a combination of a few of them. Among them, it is especially preferable that an inorganic filler is used. In addition, a shape of the non acid-reactive powder having no pore is not particularly limited, and may be any shape such as arbitral shapes such as spherical, needle-like, plate-like, ground-like and scaly-shapes may be used. There is no particular limitation on the 50% particle diameter (D50) of these of the non acid-reactive powder having no pore, but it is preferably 0.001 µm or more and 30 µm or less.

Specific examples of the inorganic filler having no pore include quartz, amorphous silica, ultrafine silica, various glasses which does not contain element which may react with an acid group (including a glass by melting method, synthetic glass by sol-gel method, a glass produced by a vapor phase reaction, and the like), silicon nitride, silicon carbide, boron carbide and the like, but is not limited thereto. The inorganic filler having no pore can be used alone or in a combination of a few kinds.

When the non acid-reactive powder having no pore is contained in the dental glass ionomer cement composition of the present invention, it is preferable that the content of the non acid-reactive powder having no pore is within a range of 0.001 % by mass or more and 20% by mass or less based on the whole composition. The dental glass ionomer cement composition of the present invention may not contain a non acid-reactive powder having no pore.

Furthermore, when the dental glass ionomer cement composition for luting of the present invention includes a paste form, a thickener can be optionally contained to such a range that various properties are not adversely affected, for the purpose of adjusting the paste property.

As the thickener which can be used in the dental glass ionomer cement composition of the present invention, any of an inorganic thickener and an organic thickener may be used. Specific examples of an inorganic thickener include fumed silica, calcium carbonate, calcium silicate, magnesium silicate, and a clay mineral such as saponite, montmorillonite, beidellite, vermiculite, sauconite, stevensite, hectorite, smectite, nekutaito and sepiolite.

Specific examples of an organic thickener include methyl cellulose, hydroxyethyl cellulose, hydroxymethyl cellulose, hydroxypropyl cellulose, carboxymethyl cellulose, sodium carboxymethyl cellulose, calcium carboxymethyl cellulose, carboxypolymethylene, sodium alginate, propylene glycol alginate ester, sodium polyacrylate, starch, starch sodium glycolate, starch phosphate ester, polyvinyl pyrrolidone, carboxyvinyl polymer, khaya gum, arabic gum, karaya gum and guar gum and xanthan gum.

The above described thickener is not limited to these, and may be used alone or in a combination of a few kinds thereof. When the thickener is contained in the dental glass ionomer cement composition of the present invention, it is preferable that the content of the thickener in the paste is 0.001 % by mass or more and 10.0 % by mass or less. The dental glass ionomer cement composition of the present invention may not contain a thickener.

In addition, the dental glass ionomer cement composition of the present invention may arbitrarily contain a polymerization inhibitor, a chain transfer agent, a discoloration inhibitor, an ultraviolet absorber, a preservative, an antibacterial agent, a colorant, a fluorescent agent, an inorganic fiber material, an organic fiber material and other conventionally known additives, as necessary.

The dental glass ionomer cement composition of the present invention may be provided in any form, such as powder material/liquid material, paste/paste or paste/liquid material, so long as the (a) acid-reactive glass powder does not coexist with the (b) polyalkenoic acid in the presence of the (c) water.

It is preferable that the dental glass ionomer cement composition of the present invention shortens the time until shaping can begin by adjusting the compound amounts of the above described components, thereby shortening treatment time. In order to shorten the treatment time, the shaping start time is preferably within 30 seconds, and more preferably within 20 seconds.

The dental glass ionomer cement composition of the present invention can be used in a wide range of applications in dental treatment, such as pit and fissure sealant, lining material and abutment construction material, in addition to being used as filling material and luting materials.

### [Examples]

Hereinafter, the present invention will be described in detail with reference to Examples and Comparative Examples. However, the present invention is not limited to these Examples. The components (a) to (d) and other components used for manufacturing the dental glass ionomer cement compositions of Examples and Comparative Examples, their abbreviations and preparing methods are as follows.

### [(a) Acid-reactive glass powder]

G1: Acid-reactive glass powder 1 (G1) (fluoroaluminosilicate glass powder, 50% particle diameter (D50): 2.5 µm)
G2: Acid-reactive glass powder 2 (G2) (fluoroaluminosilicate glass powder, 50% particle diameter (D50): 20 µm)
G3: Acid-reactive glass powder 3 (G3) (fluoroaluminosilicate glass powder, 50% particle diameter (D50): 2.5 µm)

PCA1: acrylic acid-tricarboxylic acid copolymer powder (weight average molecular weight: 80,000)
PCA2: acrylic acid-tricarboxylic acid copolymer powder (weight average molecular weight: 140,000)
PCA3: acrylic acid homopolymer powder (weight average molecular weight: 50,000)
PCA4: acrylic acid homopolymer powder (weight average molecular weight: 300,000)
PCA5: acrylic acid homopolymer powder (weight average molecular weight: 10,000)
PCA6: acrylic acid homopolymer powder (weight average molecular weight: 350,000)

### [(c) Water]

### IEW: Ion-exchanged water

### [(d) Porpus inorganic filler]

PIF1: Porous inorganic filler (SiO₂: 80 % by mass, ZrO: 20 % by mass, 50 % particle diameter (D50): 2.0 µm, pore volume: 0.08 cc/g, specific surface area: 15 m²/g)
PIF2: Porous inorganic filler (SiO₂: 80 % by mass, ZrO: 20 % by mass, 50 % particle diameter (D50): 3.0 µm, pore volume: 0.20 cc/g, specific surface area: 135 m²/g)
PIF3: Porous inorganic filler (SiO₂: 80 % by mass, ZrO: 20 % by mass, 50 % particle diameter (D50): 4.0 µm, pore volume: 0.30 cc/g, specific surface area: 200 m²/g)
PIF4: Porous inorganic filler (SiO₂: 100 % by mass 50% particle diameter (D50): 9.0 µm, pore volume: 0.80 cc/g, specific surface area: 200 m²/g)
PIF5: Surface-treated porous inorganic filler (SiO₂: 80 % by mass, ZrO: 20 % by mass, 50 % particle diameter (D50): 3.0 µm, pore volume: 0.19 cc/g, specific surface area: 133 m²/g)
PIF6: Porous inorganic filler (SiO₂: 94 % by mass, ZrO: 6 % by mass, 50 % particle diameter (D50): 2.9 µm, pore volume: 0.21 cc/g, specific surface area: 173 m²/g)
PIF7: Porous inorganic filler (SiO₂: 70 % by mass, ZrO: 30 % by mass, 50 % particle diameter (D50): 2.9 µm, pore volume: 0.19 cc/g, specific surface area: 161 m²/g)
PIF8: Porous inorganic filler (SiO₂: 65 % by mass, ZrO: 35 % by mass, 50 % particle diameter (D50): 2.9 µm, pore volume: 0.17 cc/g, specific surface area: 149 m²/g)

### [Others]

·Fuselex X: Crushed silica filler (non acid-reactive powder having no pore, 50% particle diameter (D50): 3.0 µm, pore volume: less than 0.01 cc/g)
·Aerosil R972: Spherical silica filler (thickener, primary particle diameter 16 nm, pore volume: less than 0.01 cc/g)
·TA: Tartaric acid

### [Manufacture of (a) acid-reactive glass powder]

### [Manufacture of acid-reactive glass powder 1 (G1)]

Various raw materials: silica, alumina, aluminum phosphate, sodium fluoride, and strontium carbonate (glass composition: SiO₂: 26.4 % by mass, Al₂O₃: 29.3 % by mass, SrO: 20.5 % by mass, P₂O₅: 10.9 % by mass, Na₂O: 2.5 % by mass, and F: 10.4 % by mass) were mixed and the mixed material was molten at 1400 °C in a melting furnace. The molten liquid was taken out from the melting furnace and was quenched in water to manufacture a fluoroaluminosilicate glass. The resulting fluoroaluminosilicate glass was pulverized until the 50% particle diameter (D50) became 2.5 µm to obtain acid-reactive glass powder 1 (G1). The 50% particle diameter was measured by a laser diffraction type grain size measuring apparatus (Microtrac MT3300EXII, manufactured by Microtrac Bell Co., Ltd.).

### [Manufacture of acid-reactive glass powder 2 (G2)]

Acid-reactive glass powder 2 was prepared by the same method as the acid-reactive glass powder 1 except that the 50% particle diameter was 20 µm by adjusting the pulverization time.

### [Manufacture of acid-reactive glass powder 3 (G3)]

Acid-reactive glass powder 3 was prepared by the same method as the acid-reactive glass powder 1 except that the 50% particle diameter was 25 µm by adjusting the pulverization time.

### [Manufacture of surface-treated porous inorganic filler (PIF5)]

A surface treatment liquid (total mass: 9.2 parts by mass) was prepared by mixing 0.3 part by mass of γ-methacryloyloxypropyl trimethoxysilane, 0.1 part by mass of ion-exchanged water and 8.8 parts by mass of absolute ethanol. This surface treatment liquid and 100 parts by mass of Porous inorganic filler (PIF2) were dry-mixed, and then heat-treated at 110 °C for 5 hours using a hot air dryer to obtain Porous inorganic filler (PIF5).

### [Preparation of powder material and liquid material, or first paste and second paste]

The various components were mixed in the ratios shown in Tables 1 to 5 to prepare powder materials, liquid materials, first pastes, and a second pastes .

Composition of the powder material used in Examples and Comparative examples (% by mass)

**[Table 1]**

| | | P1 | P2 | P3 | P4 | P5 | P6 | P7 | P8 | P9 | P10 |
|---|---|---|---|---|---|---|---|---|---|---|---|
| (a) Acid-reactive glass powder | G1 | 95.00 | 95.00 | 95.00 | 95.00 | 94.00 | 96.00 | 90.00 | 80.00 | 99.90 | |
| | G2 | | | | | | | | | | 95.00 |
| | G3 | | | | | | | | | | |
| (d) Porous inorganic filler | PIF1 | 5.00 | | | | 6.00 | 4.00 | 10.00 | 20.00 | 0.10 | 5.00 |
| | PIF2 | | 5.00 | | | | | | | | |
| | PIF3 | | | 5.00 | | | | | | | |
| | PIF4 | | | | 5.00 | | | | | | |
| | PIF5 | | | | | | | | | | |
| | PIF6 | | | | | | | | | | |
| | PIF7 | | | | | | | | | | |
| | PIF8 | | | | | | | | | | |
| Others | \|Fuselex X | | | | | | | | | | |
| Total | | 100.00 | 100.00 | 100.00 | 100.00 | 100.00 | 100.00 | 100.00 | 100.00 | 100.00 | 100.00 |

Composition of the powder material used in Examples and Comparative examples (% by mass)

**[Table 2]**

| | | P11 | P12 | P13 | P14 | P15 | P16 | P17 | P18 | P19 | P20 |
|---|---|---|---|---|---|---|---|---|---|---|---|
| (a) Acid-reactive glass powder | G1 | 100.00 | 70.00 | 99.95 | 95.00 | | 95.00 | 95.00 | 95.00 | 95.00 | 86.00 |
| | G2 | | | | | | | | | | |
| | G3 | | | | | 95.00 | | | | | |
| (d) Porous inorganic filler | PIF1 | | 30.00 | 0.05 | | 5.00 | | | | | 14.00 |
| | PIF2 | | | | | | | | | | |
| | PIF3 | | | | | | | | | | |
| | PIF4 | | | | | | | | | | |
| | PIF5 | | | | | | 5.00 | | | | |
| | PIF6 | | | | | | | 5.00 | | | |
| | PIF7 | | | | | | | | 5.00 | | |
| | PIF8 | | | | | | | | | 5.00 | |
| Others | Fuselex X | | | | 5.00 | | | | | | |
| Total | | 100.00 | 100.00 | 100.00 | 100.00 | 100.00 | 100.00 | 100.00 | 100.00 | 100.00 | 100.00 |

Composition of the liquid material used in Examples and Comparative examples (% by mass)

**[Table 3]**

| | | L1 | L2 | L3 | L4 | L5 | L6 | L7 | L8 | L9 | L10 | L11 |
|---|---|---|---|---|---|---|---|---|---|---|---|---|
| (b) Polyalkenoic acid | PCA1 | 42.50 | 58.50 | 30.00 | | | | 55.00 | | 52.00 | | |
| | PCA2 | | | | 42.50 | | | | | | | |
| | PCA3 | | | | | 42.50 | | | 33.50 | | | |
| | PCA4 | | | | | | 42.50 | | | | | |
| | PCA5 | | | | | | | | | | 42.50 | |
| | PCA6 | | | | | | | | | | | 42.50 |
| (c) Water | IEW | 51.00 | 35.00 | 63.50 | 51.00 | 51.00 | 51.00 | 38.50 | 60.00 | 41.50 | 51.00 | 51.00 |
| Others | TA | 6.50 | 6.50 | 6.50 | 6.50 | 6.50 | 6.50 | 6.50 | 6.50 | 6.50 | 6.50 | 6.50 |
| Total | | 100.00 | 100.00 | 100.00 | 100.00 | 100.00 | 100.00 | 100.00 | 100.00 | 100.00 | 100.00 | 100.00 |

Composition of the first paste used in Examples and Comparative examples (% by mass)

**[Table 4]**

| | | AP1 | AP2 |
|---|---|---|---|
| (a) Acid-reactive glass powder | G1 | 67.00 | 72.00 |
| | G2 | | |
| | G3 | | |
| (c) Water | IEW | 23.00 | 23.00 |
| (d) Porous inorganic filler | PIF1 | 5.00 | |
| | PIF2 | | |
| | PIF3 | | |
| | PIF4 | | |
| | PIF5 | | |
| | PIF6 | | |
| | PIF7 | | |
| | PIF8 | | |
| Others | Aerosil R972 | 5.00 | 5.00 |
| Total | | 100.00 | 100.00 |

Composition of the second paste used in Examples and Comparative examples (% by mass)

**[Table 5]**

| | | BP1 | BP2 | BP3 | BP4 |
|---|---|---|---|---|---|
| (b) Polyalkenoic acid | PCA1 | 40.00 | | 35.00 | |
| | PCA2 | | | | |
| | PCA3 | | 40.00 | | 35.00 |
| | PCA4 | | | | |
| | PCA5 | | | | |
| | PCA6 | | | | |
| (c) Water | IEW | 49.50 | 49.50 | 54.50 | 54.50 |
| Others | Aerosil R972 | 5.00 | 5.00 | 5.00 | 5.00 |
| | TA | 5.50 | 5.50 | 5.50 | 5.50 |
| Total | | 100.00 | 100.00 | 100.00 | 100.00 |

### [Dental glass ionomer cement compositions]

Dental glass ionomer cement compositions for filling or luting in which the powder material and the liquid material, or the first paste and the second paste are combined in the powder-liquid ratio or paste ratio (mass ratio) shown in Tables 6 to 9 respectively (Examples 1 to 33 and Comparative Examples 1 to 7) were evaluated for mixability, stringiness of mixed material, shaping start time and compressive strength. For Examples 1 to 16, 20, 22 to 25, 27 to 32 and Comparative Examples 1, 3 to 6, which are the compositions for filling, all of the above items were evaluated, and for Examples 17 to 19, 21, 26, 33 and Comparative Examples 2, 7 which are the compositions for luting, items other than the shaping start time were evaluated. The evaluation method is as follows.

### [Mixiability]

Under an environment of a temperature of 23 ± 1 °C and a humidity 50 ± 10 %, the powder material and the liquid material, or the first paste and the second paste, of the dental glass ionomer cement compositions of the Examples or Comparative Examples were mixed using a plastic spatula in the ratios shown in Tables 6 to 9. At this time, the time from the start of mixing was used as the base point, and the time until no remaining powder material was visually observed in the mixed material and the mixed material became homogeneous was measured. The total amount of the powder material and the liquid material, or the first paste and the second paste, was 360 mg. When the evaluation result according to the following evaluation criteria was A or B, it was determined to have good mixiability. Three evaluators each performed three times of evaluations, and the most common evaluation result was taken as the evaluation result of the measurement object.

### -Evaluation criteria-

### [Powder-liquid type]

A: Time required for the mixed material to become uniform was less than 30 seconds.
B: Time required for the mixed material to become uniform was 30 seconds or more and less than 50 seconds.
C: Time required for the mixed material to become uniform was 50 seconds or longer or the mixed material did not become uniform.

### [Paste type]

A: Time required for the mixed material to become uniform was less than 10 seconds.
B: Time required for the mixed material to become uniform was 10 seconds or more and less than 20 seconds.
C: Time required for the mixed material to become uniform was 20 seconds or longer.

### [Stringiness of mixed material]

Under an environment of a temperature of 23 ± 1 °C and a humidity 50 ± 10 %, the powder material and the liquid material, or the first paste and the second paste, of the dental glass ionomer cement compositions of the Examples or Comparative Examples were mixed using a plastic spatula in the ratios shown in Tables 6, 7, 8 and 9. The total amount of the powder material and the liquid material, or the first paste and the second paste, was 360 mg. Immediately after the end of mixing, the mixed material was filled into a plastic simulated cavity (having 4 mm x 8 mm x 2 mm and simulating a class I cavity), and the excess was scraped off to make the surface flat. Ten seconds after the end of mixing, the cylindrical tip (diameter φ1.5 mm) of a metallic instrument (MiCD instrument manufactured by SHOFU INC.) was vertically submerged 0.5 mm into the mixed material, and the instrument was immediately and gently pulled up. At this time, the degree of stringiness of the mixed material was evaluated according to the following evaluation criteria, and when the evaluation result was A or B, it was determined to have good mixed material property with little stringiness. Three evaluators each performed three evaluations, and the most common evaluation result was taken as the evaluation result of the measurement object.

### -Evaluation criteria-

A: No stringiness.
B: Slightly stringiness.
C: Significant stringiness.

### [Shaping start time]

Under an environment of a temperature of 23 ± 1 °C and a humidity 50 ± 10 %, the powder material and the liquid material, or the first paste and the second paste, of the dental glass ionomer cement compositions of the Examples or Comparative Examples were mixed using a plastic spatula in the ratios shown in Tables 6 to 9. The total amount of the powder material and the liquid material, or the first paste and the second paste, was 360 mg. Immediately after the end of mixing, the mixed material was filled into a plastic simulated cavity (having 4 mm x 8 mm x 2 mm and simulating a class I cavity), and the excess was scraped off to make the surface flat. The tip (diameter φ1.5 mm) of an instrument (MiCD instrument manufactured by SHOFU INC.) was vertically submerged 0.5 mm into the mixed material, and the instrument was immediately and gently pulled up. At this time, the time from the end of mixing was used as the base point, and the time until the stringiness of the mixed material was reduced and it was possible to perform shaping operation was measured at 10 second intervals. When the evaluation result according to the following evaluation criteria was A or B, it was determined that the shaping start time was early. Three evaluators each performed three times of evaluations, and the most common evaluation result was taken as the evaluation result of the measurement object.

### -Evaluation criteria-

A: 20 seconds after the end of mixing, the stringiness of the mixed material was reduced and shaping operations were possible.
B: 30 seconds after the end of mixing, the stringiness of the mixed material was reduced and shaping operations were possible.
C: After 40 seconds or more from the end of mixing, the stringiness of the mixed material is reduced and shaping operation was possible.

### [Compressive strength]

A compressive strength was measured by the following procedures according to ISO 9917-1:2007. Under an environment of a temperature of 23 ± 1 °C and a humidity 50 ± 10 %, the powder material and the liquid material, or the first paste and the second paste, of the dental glass ionomer cement compositions of the Examples or Comparative Examples were mixed using a plastic spatula in the ratios shown in Tables 6 to 9. The mixed material was filled into a stainless mold (cylindrical shape having an inner diameter of 4 mm and a height of 6 mm), and then allowed to stand in a thermo-hygrostat chamber at a temperature of 37 °C and a humidity of 90% or more. After standing for 1 hour, the set product was removed from the mold and used as a test specimen. After immersing the test specimen in ion-exchanged water at 37 °C for 24 hours from the end of mixing, the compressive strength of the test specimen was measured at a crosshead speed of 1 mm/min using an Instron universal testing machine (model: 5567A). When the evaluation result according to the following evaluation criteria was A or B, it was determined to have good mechanical characteristic. Five evaluators each performed five times of evaluations, and the most common evaluation result was taken as the evaluation result of the measurement object.

### -Evaluation criteria-

### [Composition for filling]

A: Compressive strength was 220 MPa or more.
B: Compressive strength was 200 MPa or more and less than 220 MPa.
C: Compressive strength was less than 200 MPa.

### [Compositions for luting]

A: Compressive strength was 160 MPa or more
B: Compressive strength was 140 MPa or more and less than 160 MPa.
C: Compressive strength was less than 140 MPa.

The results of the evaluation of each composition in the examples and comparative examples according to the above test method are shown in Tables 6 to 9.

Evaluation results of Examples 1 to 11

**[Table 6]**

| | | Example 1 | Example 2 | Example 3 | Example 4 | Example 5 | Example 6 | Example 7 | Example 8 | Example 9 | Example 10 | Example 11 |
|---|---|---|---|---|---|---|---|---|---|---|---|---|
| Type | | Powder/Liquid | Powder/Liquid | Powder/Liquid | Powder/Liquid | PowderLiquid | Powder/Liquid | Powder/Liquid | Powder/Liquid | Powder/Liquid | Powder/Liquid | Powder/Liquid |
| Powder material or First paste | | P 1 | P2 | P3 | P4 | P5 | P6 | P7 | P8 | P9 | P9 | P1 |
| Liquid material or Second paste | | L1 | L1 | L1 | L1 | L1 | L1 | L1 | L1 | L1 | L2 | L3 |
| Powder-Liquid Ratio (w/w) or Paste Ratio (w/w) | | 3.0/1.0 | 3.0/1.0 | 3.0/1.0 | 3.0/1.0 | 3.0/1.0 | 3.0/1.0 | 3.0/1.0 | 3.0/1.0 | 3.0/1.0 | 4.0/1.0 | 3.0/1.0 |
| Use | | Filling | Filling | Filling | Filling | Filling | Filling | Filling | Filling | Filling | Filling | Filling |
| (a) Acid-reactive glass powder (% by mass) | G1 | 71.250 | 71.250 | 71.250 | 71.250 | 70.500 | 72.000 | 67.500 | 60.000 | 74925 | 79920 | 71.250 |
| | G2 | | | | | | | | | | | |
| | G3 | | | | | | | | | | | |
| (b) Polyalkenoic acid (% by mass) | PCA1 | 10.625 | 10.625 | 10.625 | 10 625 | 10.625 | 10 625 | 10.625 | 10.625 | 10.625 | 11700 | 7.500 |
| | PCA2 | | | | | | | | | | | |
| | PCA3 | | | | | | | | | | | |
| | PCA4 | | | | | | | | | | | |
| | PCA5 | | | | | | | | | | | |
| | PCA6 | | | | | | | | | | | |
| (c) Water (% by mass) | IEW | 12750 | 12750 | 12750 | 12750 | 12750 | 12750 | 12750 | 12750 | 12750 | 7.000 | 15.875 |
| (d) Porous inorganic filler (% by mass) | PIF1 | 3750 | | | | 4500 | 30.00 | 7 500 | 15.000 | 0.075 | 0.080 | 3750 |
| | PIF2 | | 3750 | | | | | | | | | |
| | PIF3 | | | 3750 | | | | | | | | |
| | PIF4 | | | | 3750 | | | | | | | |
| | PIF5 | | | | | | | | | | | |
| | PIF6 | | | | | | | | | | | |
| | PIF7 | | | | | | | | | | | |
| | PIF8 | | | | | | | | | | | |
| Others (% by mass) | Fuselex X | | | | | | | | | | | |
| | Aerosil R972 | | | | | | | | | | | |
| | TA | 1 625 | 1 625 | 1 625 | 1 625 | 1 625 | 1 625 | 1 625 | 1 625 | 1 625 | 1.300 | 1 625 |
| Total (% by mass) | | 100.000 | 100.000 | 100.000 | 100.000 | 100.000 | 100.000 | 100.000 | 100.000 | 100.000 | 100.000 | 100.000 |
| Mixability | | A | A | A | A | A | A | A | A | A | B | A |
| Stringiness of mixed material | | A | A | A | A | A | A | A | A | B | A | A |
| Shaping start time (sec) | | 20 | 20 | 20 | 20 | 20 | 20 | 20 | 30 | 30 | 20 | 30 |
| Evaluation | | A | A | A | A | A | A | A | B | B | A | B |
| Compressive strength (Mpa) | | 250 | 244 | 240 | 238 | 247 | 252 | 230 | 218 | 248 | 264 | 207 |
| Evaluation | | A | A | A | A | A | A | A | B | A | A | B |

Evaluation results of Examples 12 to 22

**[Table 7]**

| | | Example 12 | Example 13 | Example 14 | Example 15 | Example 16 | Example 17 | Example 18 | Example 19 | Example 20 | Example 21 | Example 22 |
|---|---|---|---|---|---|---|---|---|---|---|---|---|
| Type | | PowderLiaud | Powder/Liquid | PowderLiaud | Powder/Liquid | PowderLiaud | Powder/Liquid | Powder/Liauₗd | PowderLiaud | Paste | Paste | Powder/Liquid |
| Powder material or First paste | | P1 | P1 | P1 | P10 | P1 | P1 | P1 | P1 | AP1 | AP1 | P15 |
| Liquid material or Second paste | | L4 | L5 | L6 | L1 | L7 | L8 | L1 | L9 | BP1 | BP2 | L1 |
| Powder-Liquid Ratio (w/w) or Paste Ratio (w/w) | | 3.0/1.0 | 3.0/1.0 | 3.0/1.0 | 3.0/1.0 | 3.0/1.0 | 2.0/1.0 | 12/10 | 1.6/1.0 | 3.0/1.0 | 2.0/1.0 | 3.0/1.0 |
| Use | | Filling | Filling | Filling | Filling | Filling | Luting | Luting | Luting | Filling | Luting | Filling |
| (a) Acid-reactive glass powder (% by mass) | G1 | 71.250 | 71.250 | 71.250 | | 71.250 | 63 333 | 51.818 | 58461 | 50.250 | 44.667 | |
| | G2 | | | | 71.250 | | | | | | | |
| | G3 | | | | | | | | | | | 71.250 |
| (b) Polyalkenoic acid (% by mass) | PCA1 | | | | 10 625 | 13750 | | 19.318 | 20000 | 10.000 | | 10.625 |
| | PCA2 | 10.625 | | | | | | | | | | |
| | PCA3 | | 10.625 | | | | 11167 | | | | 13.333 | |
| | PCA4 | | | 10.625 | | | | | | | | |
| | PCA5 | | | | | | | | | | | |
| | PCA6 | | | | | | | | | | | |
| (c) Water (% by mass) | IEW | 12750 | 12750 | 12750 | 12750 | 9.625 | 20.000 | 23 182 | 15 962 | 29625 | 31834 | 12750 |
| (d) Porous inorganic filler (% by mass) | PIF1 | 3750 | 3750 | 3750 | 3750 | 3750 | 3.333 | 2727 | 3 077 | 3750 | 3 333 | 3750 |
| | PIF2 | | | | | | | | | | | |
| | PIF3 | | | | | | | | | | | |
| | PIF4 | | | | | | | | | | | |
| | PIF5 | | | | | | | | | | | |
| | PIF6 | | | | | | | | | | | |
| | PIF7 | | | | | | | | | | | |
| | PIF8 | | | | | | | | | | | |
| Others (% by mass) | Fuselex X | | | | | | | | | | | |
| | Aerosil R972 | | | | | | | | | 5.000 | 5.000 | |
| | TA | 1 625 | 1 625 | 1 625 | 1 625 | 1 625 | 2.167 | 2 955 | 2500 | 1.375 | 1833 | 1 625 |
| Total (% by mass) | | 100.000 | 100.000 | 100.000 | 100.000 | 100.000 | 100.000 | 100.000 | 100.000 | 100.000 | 100.000 | 100.000 |
| Mixability | | A | A | B | A | A | A | A | A | A | A | A |
| Stringiness of mixed material | | A | A | A | A | B | A | A | B | B | B | A |
| Shaping start time (sec) | | 20 | 20 | 20 | 20 | 30 | | | | 30 | | 20 |
| Evaluation | | A | A | A | A | B | - | - | - | B | - | A |
| Compressive strength (Mpa) | | 272 | 208 | 274 | 240 | 255 | 167 | 142 | 155 | 222 | 162 | 201 |
| Evaluation | | A | B | A | A | A | A | B | B | A | A | B |

Evaluation results of Examples 23 to 33

**[Table 8]**

| | | Example 23 | Example 24 | Example 25 | Example 26 | Example 27 | Example 28 | Example 29 | Example 30 | Example 31 | Example 32 | Example 33 |
|---|---|---|---|---|---|---|---|---|---|---|---|---|
| Type | | PowderLiaud | Powder/Liquid | Powder/Liquid | Powder/Liquid | Powder/Liquid | Powder/Liquid | Powder/Liquid | Powder/Liquid | Powder/Liauₗd | Paste | Paste |
| Powder material or Frst paste | | P9 | P1 | P1 | P1 | P16 | P17 | P18 | P19 | P20 | AP1 | AP1 |
| Liquid material or Second paste | | L2 | L10 | L11 | L9 | L1 | L1 | L1 | L1 | L1 | BP3 | BP4 |
| Powder-Liquid Ratio (w/w) or Paste Ratio (w/w) | | 4.2/1.0 | 3.0/1.0 | 3.0/1.0 | 1.2/1.0 | 3.0/1.0 | 3.0/1.0 | 3.0/1.0 | 3.0/1.0 | 3.0/1.0 | 3.0/1.0 | 2.0/1.0 |
| Use | | Filling | Filling | Filling | Luting | Filling | Filling | Filling | Filling | Filling | Filling | Luting |
| (a) Acid-reactive glass powder (% by mass) | G1 | 80.688 | 71.250 | 71.250 | 51 818 | 71.250 | 71.250 | 71.250 | 71.250 | 64.500 | 50.250 | 44.667 |
| | G2 | | | | | | | | | | | |
| | G3 | | | | | | | | | | | |
| (b) Polyalkenoic acid (% by mass) | PCA1 | 11 250 | | | 23 636 | 10.625 | 10 625 | 10.625 | 10.625 | 10.625 | 8.750 | |
| | PCA2 | | | | | | | | | | | |
| | PCA3 | | | | | | | | | | | 11.667 |
| | PCA4 | | | | | | | | | | | |
| | PCA5 | | 10.625 | | | | | | | | | |
| | PCA6 | | | 10.625 | | | | | | | | |
| (c) Water (% by mass) | IEW | 6731 | 12750 | 12750 | 18.864 | 12750 | 12750 | 12750 | 12750 | 12750 | 30.875 | 33 500 |
| (d) Porous inorganic filler (% by mass) | PIF1 | 0.081 | 3750 | 3750 | 2727 | | | | | 10500 | 3750 | 3.333 |
| | PIF2 | | | | | | | | | | | |
| | PIF3 | | | | | | | | | | | |
| | PIF4 | | | | | | | | | | | |
| | PIF5 | | | | | 3750 | | | | | | |
| | PIF6 | | | | | | 3750 | | | | | |
| | PIF7 | | | | | | | 3.750 | | | | |
| | PIF8 | | | | | | | | 3750 | | | |
| Others (% by mass) | Fuselex X | | | | | | | | | | | |
| | R972 | | | | | | | | | | 5 000 | 5.000 |
| | Aerosil TA | 1.250 | 1 625 | 1 625 | 2955 | 1 625 | 1 625 | 1 625 | 1 625 | 1 625 | 1.375 | 1833 |
| Total (% by mass) | | 100.000 | 100.000 | 100.000 | 100.000 | 100.000 | 100.000 | 100.000 | 100.000 | 100.000 | 100.000 | 100.000 |
| Mixability | | B | A | B | A | A | A | A | A | A | A | A |
| Stringiness of mixed material | | A | A | A | B | A | A | A | A | A | B | B |
| Shaping start time (sec) | | 20 | 20 | 20 | | 20 | 20 | 20 | 20 | 30 | 30 | |
| Evaluation | | A | A | A | - | A | A | A | A | B | B | - |
| Compressive strength (Mpa) | | 266 | 203 | 275 | 152 | 248 | 249 | 251 | 249 | 216 | 218 | 155 |
| Evaluation | | A | B | A | B | A | A | A | A | B | B | B |

Evaluation results of Comparative Examples 1 to 7

**[Table 9]**

| | | | Comparative Example 1 | Comparative Example 2 | Comparative Example 3 | Comparative Example 4 | Comparative Example 5 | Comparative Example 6 | Comparative Example 7 |
|---|---|---|---|---|---|---|---|---|---|
| Type | | | Powder/Liquid | Powder/Liquid | Powder/Liquid | Powder/Liquid | Powder/Liquid | Paste | Paste |
| Powder material or First paste | | | P11 | P11 | P12 | P13 | P14 | AP2 | AP2 |
| Liquid material or Second paste | | | L1 | L8 | L1 | L1 | L1 | BP1 | BP2 |
| Powder-Liquid Ratio (w/w) or Paste Ratio (w/w) | | | 3.0/1.0 | 2.0/1.0 | 3.0/1.0 | 3.0/1.0 | 3.0/1.0 | 3.0/1.0 | 2.0/1.0 |
| Use | | | Filling | Luting | Filling | Filling | Filling | Filling | Luting |
| | (a) Acid-reactive glass powder (% by mass) | G1 | 75.000 | 66.666 | 52.500 | 74.962 | 71.250 | 54.000 | 48.000 |
| | | G2 | | | | | | | |
| | | G3 | | | | | | | |
| | (b) Polyalkenoic acid (% by mass) | PCA1 | 10.625 | | 10.625 | 10.625 | 10.625 | 10.000 | |
| | | PCA2 | | | | | | | |
| | | PCA3 | | 11.167 | | | | | 13.333 |
| | | PCA4 | | | | | | | |
| | | PCA5 | | | | | | | |
| | | PCA6 | | | | | | | |
| | (c) Water (% by mass) | IEW | 12.750 | 20.000 | 12.750 | 12.750 | 12.750 | 29.625 | 31.834 |
| | (d) Porous inorganic filler (% by mass) | PIF1 | | | 22.500 | 0.038 | | | |
| | | PIF2 | | | | | | | |
| | | PIF3 | | | | | | | |
| | | PIF4 | | | | | | | |
| | | PIF5 | | | | | | | |
| | | PIF6 | | | | | | | |
| | | PIF7 | | | | | | | |
| | | PIF8 | | | | | | | |
| | Others (% by mass) | Fuselex X | | | | | 3.750 | | |
| | | Aerosil R972 | | | | | | 5.000 | 5.000 |
| | | TA | 1.625 | 2.167 | 1.625 | 1.625 | 1.625 | 1.375 | 1.833 |
| Total (% by mass) | | | 100.000 | 100.000 | 100.000 | 100.000 | 100.000 | 100.000 | 100.000 |
| Mixability | | | A | A | A | A | A | A | A |
| Stringiness of mixed material | | | C | C | A | C | C | C | C |
| Shaping start time (sec) | | | 40 | | 30 | 40 | 40 | 40 | |
| Evaluation | | | C | | B | C | C | C | |
| Compressive strength (Mpa) | | | 248 | 165 | 196 | 249 | 251 | 221 | 161 |
| Evaluation | | | A | A | c | A | A | A | A |

### <Examples 1 to 16, 20, 22 to 25, 27 to 32>

In Examples 1 to 16, 20, 22 to 25, 27 to 32, there was little stringiness immediately after mixing, and it was possible to perform shaping operations soon after filling the simulated cavity. Furthermore, good mixability and mechanical characteristic were exhibited, and thus these Examples had desirable properties as a dental glass ionomer cement composition for filling.

### <Examples 17 to 19, 21, 26, 33>

In Examples, 17 to 19, 21, 26, 33, there was little stringiness from immediately after mixing. Furthermore, good mixability and mechanical characteristic were exhibited, and thus these Examples had desirable properties as a dental glass ionomer cement composition for luting.

### <Comparative Example 1>

The dental glass ionomer cement composition for filling of Comparative Example 1 did not contain the (d) porous inorganic filler. As a result of evaluating Comparative Example 1, significant stringiness was observed in immediately after mixing and a long time was required until shaping operation became possible.

### <Comparative Example 2>

The dental glass ionomer cement composition for luting of Comparative Example 2 did not contain the (d) porous inorganic filler. As a result of evaluating Comparative Example 2, significant stringiness was observed in immediately after mixing.

### <Comparative Example 3>

The dental glass ionomer cement composition for filling of Comparative Example 3 has a high content of the (d) porous inorganic filler. As a result of evaluating Comparative Example 3, the compressive strength was low.

### <Comparative Example 4>

The dental glass ionomer cement composition for filling of Comparative Example 4 has a low content of the (d) porous inorganic filler. As a result of evaluating Comparative Example 4, significant stringiness was observed in immediately after mixing and a long time was required until shaping operation became possible.

### <Comparative Example 5>

The dental glass ionomer cement composition for filling of Comparative Example 5 contains a non acid-reactive powder having no pores instead of the (d) porous inorganic filler. As a result of evaluating Comparative Example 5, significant stringiness was observed in immediately after mixing and a long time was required until shaping operation became possible.

### <Comparative Example 6>

The dental glass ionomer cement composition for filling of Comparative Example 6 did not contain the (d) porous inorganic filler. As a result of evaluating Comparative Example 6, significant stringiness was observed in immediately after mixing and a long time was required until shaping operation became possible.

### <Comparative Example 7>

The dental glass ionomer cement composition for luting of Comparative Example 7 did not contain the (d) porous inorganic filler. As a result of evaluating Comparative Example 7, significant stringiness was observed in immediately after mixing.

With respect to the use of substantially any plural and/or singular terms herein, those having skill in the art can translate from the plural to the singular and/or from the singular to the plural as is appropriate to the context.

Although the description herein has been given with reference to the drawings and embodiments, it should be noted that those skilled in the art may make various changes and modifications on the basis of this invention without difficulty. Accordingly, any such changes and modifications are intended to be included in the scope of the embodiments.

### [Industrial applicability]

The dental glass ionomer cement composition of the present invention can be suitably used in dental treatments such as filling dental cavities, luting dental prosthesis devices such as lining layers or relinings, crowns, inlays and bridges to tooth substance, pit and fissure sealant, preventive coating of tooth surfaces, and abutment construction.

## Claims

1. A dental glass ionomer cement composition comprising
(a) acid-reactive glass powder,
(b) polyalkenoic acid,
(c) water, and
(d) porous inorganic filler: 0.075 % by mass or more and 15 % by mass or less, wherein
the center portion of the (d) porous inorganic filler is an inorganic particle consisting of only silicon dioxide or consisting of silicon dioxide and an oxide containing one or more metal elements.

2. The dental glass ionomer cement composition according to claim 1, wherein
the (d) porous inorganic filler has a 50 % particle diameter (D50) within the range of 0.1 µm or more and 10 µm or less, a pore volume within the range of 0.01 cc/g or more and 1.00 cc/g or less, and a specific surface area within the range of 5 m²/g or more and 500 m²/g or less.

3. The dental glass ionomer cement composition according to claim 1 or 2, comprising,
(a) an acid-reactive glass powder: 44 % by mass or more and 80 % by mass or less,
(b) a polyalkenoic acid: 7.5 % by mass or more and 20 % by mass or less,
(c) water: 7 % by mass or more and 32 % by mass or less, and
(d) porous inorganic filler: 0.075 % by mass or more and 15 % by mass or less.

4. The dental glass ionomer cement composition according to claim 1 or 2, wherein,
shaping start time of the dental glass ionomer cement composition is within 30 seconds.

5. The dental glass ionomer cement composition according to claim 3, wherein,
shaping start time of the dental glass ionomer cement composition is within 30 seconds.
